# EUROPEAN PATENT SPECIFICATION

(11) **EP 0 728 448 B1**
(45) Date of publication and mention of the grant of the patent: **14.06.2000**
(21) Application number: 94830574.3
(22) Date of filing: 13.12.1994
(51) Int. Cl.: A61F 2/30, A61F 2/34

(54) **Cup for a hip joint prosthesis**
Verankerungsschale einer Hüftgelenkspfanne
Coquille de fixation pour prothèse de hanche

(43) Date of publication of application: 28.08.1996
(73) Proprietor: Garosi, Piero, I-50053 Empoli (IT)
(72) Inventor: Garosi, Piero, I-50053 Empoli (IT)
(74) Representative: Mannucci, Gianfranco, Dott.-Ing.

(56) References cited:
- EP-A- 0 483 023
- EP-A- 0 499 475
- EP-A- 0 668 063
- FR-A- 2 633 823
- FR-A- 2 682 588
- US-A- 5 314 488

## Description

The present invention relates to a cup for a hip joint prosthesis according to the preamble of Claim 1.

In the surgical reconstruction of the acetabular cavity of the hip joint, cups comprising an outer metal calotte and an inner portion, normally of polyethylene, are employed. The outer metal calotte is implanted in the suitably prepared acetabular cavity in the ilium. Once the metal calotte has been inserted in the acetabular cavity, an insert, which is normally of polyethylene, is fitted inside the calotte. The calotte and the insert together form the artificial acetabulum in which the head of the femoral component of the prosthesis is then placed.

The calotte can be fixed in the acetabular cavity by various methods, and it is on the basis of these that the type of cup is usually classified.

The earliest method involved the use of a suitable cement which, when applied between the outer surface of the calotte and the surface of the acetabular cavity, ensured the stability of the cup.

In more recent times cups have been developed in which the calotte is implanted in the acetabular cavity with the aid of screw means. Some of these cups have a thread on the spherical outer surface of the metal calotte which is used to anchor it in the bone by turning it around its axis.

In other kinds of prosthesis, the metal calotte of the cup is anchored in the acetabular cavity by means of variously arranged projections on the outer surface of the calotte. FR-A-2,651,675 for example, discloses such a cup having two series of teeth extending along two great circular lines of the metal calotte from an intermediate point towards the apex.

FR-A-2,649,005 discloses a prosthesis in which the metal calotte of the cup is anchored by means of screws passing through through holes formed, in various positions, over the spherical surface of the calotte.

FR-A-2,598,908 discloses a cotyloidal prosthesis that is anchored in the acetabular cavity by means of screws which are inserted through circular through holes formed in the thickness of the metal calotte. The metal calotte is also provided with bridges which extend parallel to the axis of the cup and which, when tapped into the acetabular cavity, give an initial degree of stability against rotation, thus allowing subsequent anchoring operations to be performed with the screws.

The same method of anchoring as that disclosed in FR-A-2,598,908 is also adopted in the prosthesis of EP-A-0 483 023, in which the initial stability is entrusted to small projections formed close to the edge of the metal calotte. The purchase provided by these projections is sufficient to prevent the calotte rotating during the subsequent operation of unscrewing from the calotte the tool used to fit the calotte itself, and that of applying the anchoring screws. The latter screws, which are introduced through through holes variously arranged around the calotte, ensure the definitive stability of the calotte, prior to the insertion of the inner plastic part. Here again, therefore, the final stability of the cup is ensured by fastening screws introduced through through holes formed in the calotte.

The nearest prior art is disclosed in document EP-A-0 668 063 which falls under Article 54(3) EPC.

All the prostheses disclosed in the abovementioned publications comprise a metal calotte containing at least one fissure that extends from the apex region to the edge in order to give the calotte a degree of elasticity.

The use of through screws for the implanting of cups presents many disadvantages. Thus, if the calotte is not made absolutely immobile in the acetabular cavity, even the slightest movement of the calotte will produce very large concentrated loads on the anchoring screws, possibly sufficient to break them, which will have negative impact on stability, or else will create osteolytic regions around the screws leading to loss of the initial stability of the implant. Also, the very fact of having to fit anchoring screws during the surgical operation makes the use of these cups more difficult and lengthens the operating time.

The object of the present invention is to provide a cup that does not have the drawbacks of conventional prostheses as discussed above.

The basis of the invention is the recognition of the fact that if the outer, normally metal, calotte has sufficient elasticity, and if it is provided with tongues on the edge of the calotte, on either side of the fissure and of sufficient radial length, the calotte can be anchored in the bone without using auxiliary screws. The invention therefore suggests the making of a cup of the kind referred to at the outset, in which
- the wall thickness of the calotte is substantially uniform throughout said calotte, and comprised between 1,5 and 3 mm;
- the size of the calotte and the thickness of the calotte are such as to produce an elastic deformation of contraction as the calotte is tapped into the acetabular cavity, followed by expansion to the original size when the inner portion is inserted therein; and
- said tongues have such a radial extension that when the calotte has been tapped home into the acetabular cavity and the inner portion has been inserted into said calotte, the cup is firmly anchored to the bone.

Surprisingly, and contrary to received opinion among those skilled in the art, it is possible with these structural features to make the cup stable without using cements and without the need for screws.

To achieve sufficient cup stability, the calotte must be sufficiently elastic. For this purpose the wall thickness of the calotte is substantially uniform throughout, typically approximately 2 mm, and more generally between 1.5 and 3 mm.

In order to ensure that the tongues have ideal purchase in the bone, the calotte is preferably a complete hemisphere, and the tongues are positioned so as to extend from the base edge of the calotte, along a respective great circular line, towards the apex of the calotte. In contrast to conventional calottes, which have tongue projections on the calotte surface but away from the edge, this positioning ensures improved purchase.

Each of said tongues preferably extends along the outer surface of the calotte for at least about one third of the height of the calotte and comprises an approximately straight upper edge at an inclined angle to the axis of the calotte. Said upper edge preferably forms an angle of between approximately 45° and approximately 65° with the polar axis (A-A), and in particular of approximately 55°. This guarantees ideal penetration and purchase in the bone.

Advantageously, said tongues project radially from the outer surface of said calotte by more than twice the thickness of the wall of the calotte. Their size is such as to enable them to be anchored even where there is porous bone in the antero- and posteroinferior region of the acetabulum.

Other advantages characteristic of the cup according to the invention will be described below and are indicated in the attached dependent claims.

A better understanding of the invention would be provided by the description and the accompanying drawing, which latter shows a practical and non-restricting example of the invention. In the drawing:
Fig. 1 shows a view from below of the calotte of a cup according to the present invention;
Fig. 2 shows a section on II-II shown in Fig. 4;
Fig. 3 shows a side view on III-III shown in Fig. 2;
Fig. 4 shows a plan view on IV-IV shown in Fig. 2;
Figs. 5 and 6 show two enlarged details from Figs. 1 and 2;
Fig. 7 shows a plan view on VII-VII shown in Fig. 8 of a polyethylene insert for insertion in the calotte of Figs. 1 to 6; and
Fig. 8 shows a section on VIII-VIII shown in Fig. 7.

The outer calotte and the inner plastic portion are not drawn to the same scale.

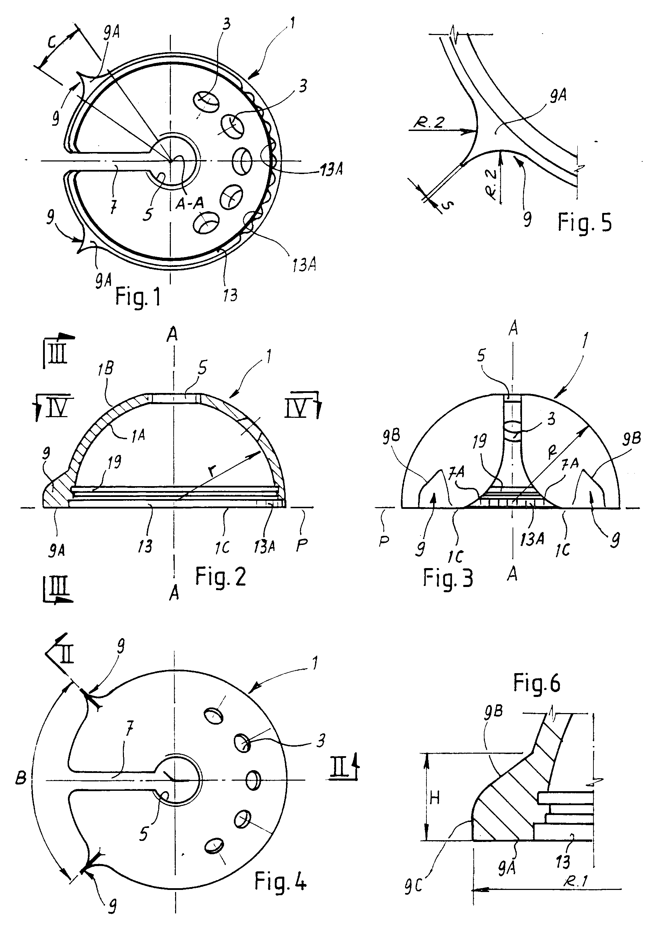

With reference to Figs. 1 to 6, the calotte 1 of the cup according to a preferred embodiment of the invention will be described first. This calotte is a complete hemisphere and its wall is of substantially constant thickness, except for a groove around its edge for the anchoring of the inner polyethylene portion. The calotte comprises an inner surface 1A of radius r and an outer surface 1B of radius R. The inner and outer surfaces are approximately concentric so that the wall thickness of the calotte is generally constant and equal to the difference between the two radii R and r. This thickness is approximately 2 mm. The hemispherical wall of the calotte 1 contains through holes 3 for the use, if necessary of screws, in certain cases. However, as will be clear from the following description, these screws are not usually necessary to achieve cup stability, which is guaranteed by the morphological structure and by the characteristics of elasticity of the calotte 1.

At the apex of the calotte is a threaded hole 5 for the positioning tool, which has a threaded head which is screwed into the thread of the hole 5. From the hole 5 there extends a fissure 7 that reaches to the edge 1C of the calotte 1, at which point the edges (which are parallel with each other for almost the whole length of the fissure 7) of the fissure 7 run into arcuate portions 7A (Fig. 3) of suitable radius.

On either side of the fissure 7 are two tongues 9 arranged symmetrically with respect to this fissure and separated by an angle B of approximately 90°. Each tongue lies in a plane containing the axis A-A of the calotte. In addition, each tongue 9 has a lower surface 9A lying on the plane marked p, at right angles to the axis A-A, in which the edge 1C of the calotte lies.

The tongues 9 extend some considerable way both axially and radially relative to the outer surface 1B of the calotte 1. In particular (see Figs. 5 and 6), in one possible embodiment of a calotte having dimensions of for example R = 25 mm and r = 23 mm, the tongue dimensions are as follows:
R1 = 30 mm
H = 9 mm
R2 = 5 mm
s = 0.4 mm
   and comprise an upper edge 9B, which forms an angle of approximately 55° with the axis A-A of the calotte 1 and meets the edge 9C with a radius of curvature of approximately 4.5 mm. The edge 9C is approximately parallel to the axis A-A, i.e. slightly inclined, by approximately 1-2°, relative to said axis.

The dimensions of the tongues and the thickness of the calotte remain approximately constant as the diameter varies. The diameter may typically be between 45 and 65 mm. The shape of the tongues enables the tongues to be very thick where they join the spherical surface of the calotte 1. As indicated in Fig. 1, in the case of a calotte having R = 25 mm, the tongues 9 have an angle of aperture C, relative to the centre of the calotte, of approximately 20°. More generally, the angle C may be between 15 and 25° approximately.

The calotte 1 may be made in any of the materials suitable for the purpose. In particular a titanium alloy Ti-6A1-4V ELI which has good mechanical characteristics and high elasticity (Young's modulus of approximately 113,000 MPa) can be used. The outer surface 1B of the calotte 1 is treated, in a manner known per se, by a plasma-spray process which consists in coating the outside with metal ions of pure titanium. This treatment promotes the adhesion of the bone tissues to the surface of the calotte after implantation.

Close to the edge 1C of the calotte 1 is a first annular groove 13 with a series of undulating teeth 13A which engage with a corresponding series of undulating depressions 15A formed in a ring 15 around the inner portion 17, which is of polyethylene or some equivalent material and is to be inserted in the calotte 1 once the latter has been implanted in the acetabular cavity. The undulating teeth and the undulating depressions define a shape fit that allows the two portions 1, 17 to fit together in a relative angular position selectable from among a plurality of possible angular positions. This permits a certain freedom of positioning of the calotte 1, allowing the tongues 9 to be implanted wherever the bone is strongest, while the inner portion 17 can be fitted at any necessary angle for correcting possible tendencies to dislocation.

In the inner surface 1A of the calotte 1 there is also a second annular groove 19 into which there is snap-fitted an elastic annular projection 21 formed on the outer surface 17B of the inner acetabular portion 17 of the prosthesis. The elastic annular projection 21 is defined by two consecutive annular grooves 23, 25 formed on the outer surface 17B. Pushing the inner portion 17 into the calotte 1 causes the annular projection 21 to enter the groove 19, thereby locking the inner portion 17, forming the artificial acetabulum 17A, in the calotte 1 anchored to the ilium.

The special system of anchoring the inner portion 17 to the calotte 1 makes it possible to avoid the presence of an external inward flange on the calotte 1, an element which is necessary in some kinds of prosthesis belonging to the prior art. The outer surface 1B of the calotte 1 can thus extend uninterrupted as far as the edge 1C, and this enables tongues 9 to be formed in alignment with said edge 1C.

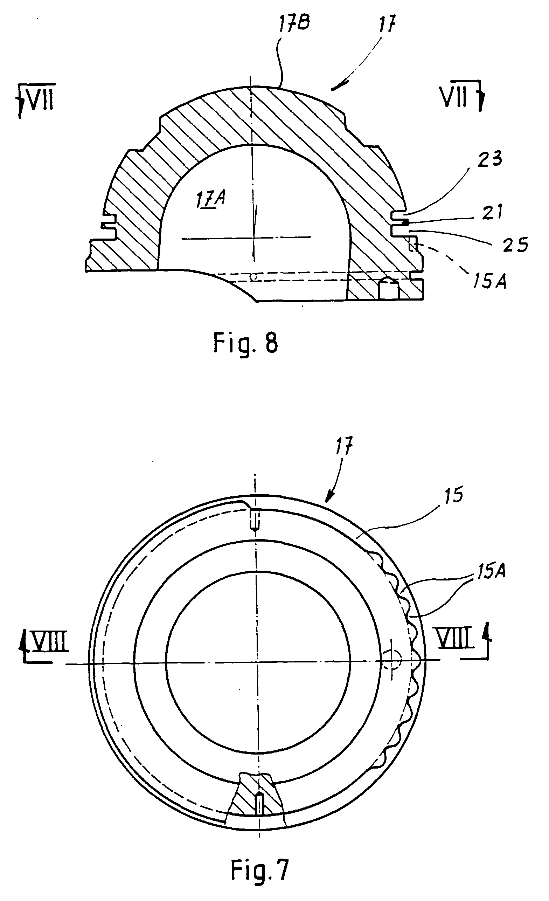

The inner portion 17 illustrated in Figs. 7 and 8 is of the asymmetrical kind used in certain pathological conditions, but it will be obvious that (as known to those skilled in the art) this portion may be of a variety of shapes and can be either symmetrical or even more asymmetrical than that illustrated.

The fitting of the cup described above is done as follows: the acetabular bone is first prepared using an acetabular reamer whose radius should be equal to the external radius R of the acetabular calotte or cup 1, after which the calotte is tapped into the seat using appropriate instruments of a kind known per se. During this stage the presence of the tongues 9 causes an elastic radial contraction of the calotte, because as tapping proceeds the tongues meet the surface of the bone of the cavity previously prepared by the surgeon. The subsequent forced insertion of the polyethylene portion 17 into the calotte 1 causes the calotte to expand radially and elastically and this further increases the initial stability of the implant. The size and shape of the tongues 9 guarantee stability whether the bone is very porous or very sclerotic, although it will be understood that the angular position of the calotte 1 can be selected from within an angular interval to suit the condition of the bone.

### Tests of elasticity

In order to verify the functionality and advantages of the cup according to the present invention as compared with conventional cups, comparative elasticity tests were carried out. A calotte 1 of a cup according to the present invention, with a nominal diameter of 58 mm, was compared with a calotte of a cup of the same nominal diameter made according to EP-A-0483023 and sold with the name Mont Blanc®, model MB 4830058®.

The purpose of the elasticity tests was to see how the shape of two cups of the same material and apparently similar form would be such as to distinguish two different elastic behaviours.

The instrumentation used in the test consisted of:
- a metal support for the calotte of the cup, consisting in an alluminium block having a sent for the calotte;
- two pins, one for applying the force to the calotte and one for reading the force. The two pins were inserted into the calotte supporting block;
- a piezoelectric load cell manufactured by DS Europa, model 546Q capable of measuring up to 220 kg;
- a signal conditioner from DS Europa, model AN 331; and
- an electronic gauge calibrated in one-hundredths.

The method adopted for the test was as follows: the calotte was placed on the metal support in contact with the force reading pin and at the same time the opposing pin was screwed in, bringing it into contact with the calotte without applying any force (the signal conditioner must not yet give any signal). Once the instrumentation has been set, a load is applied by acting on the moveable screwed pin in such a way as to create load conditions increasing by intervals of 5 kg. The following parameters were then read off:
- external diameter of the calotte at right angles to the fissure of each sample;
- least width of fissure before test commences (with no load);
- decreasing distance between the two sides of the fissure as load increases; and
- external diameter of the calotte after the test.

Following the method described above and comparing a single sample of a calotte according to EPA0 483 923 (denoted M in the tables below) with three different samples of the calotte of the cup according to the present invention (denoted G1, G2, G3), all with a nominal diameter of 58 mm, the results shown in the following four tables were obtained:

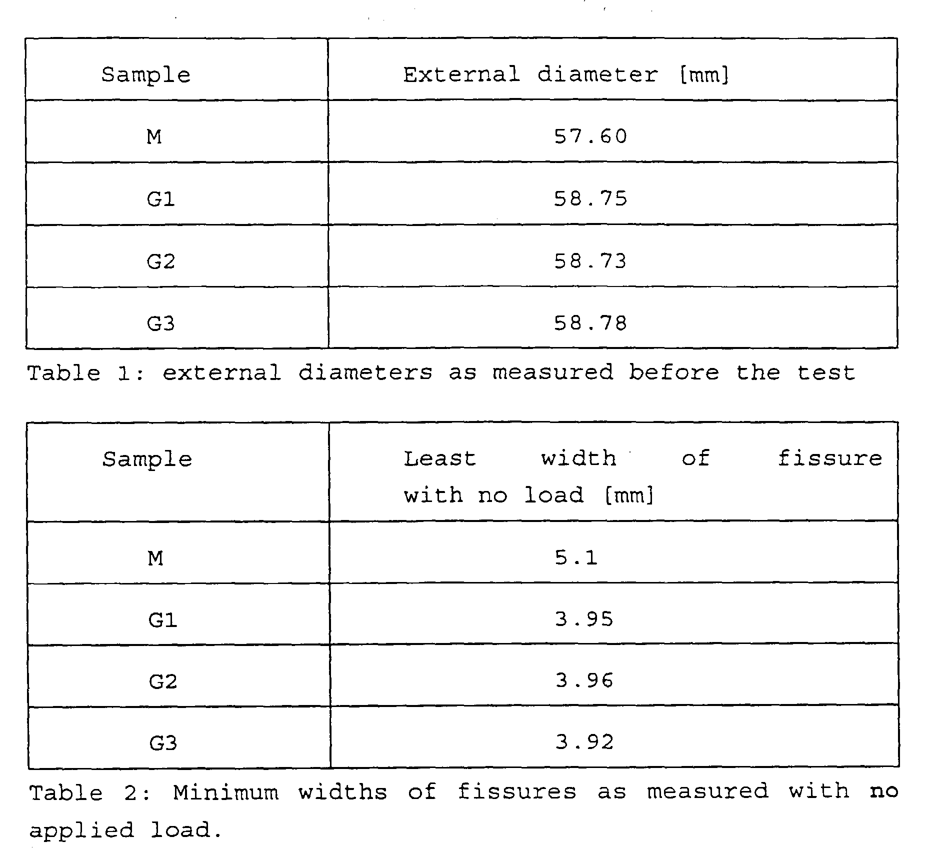

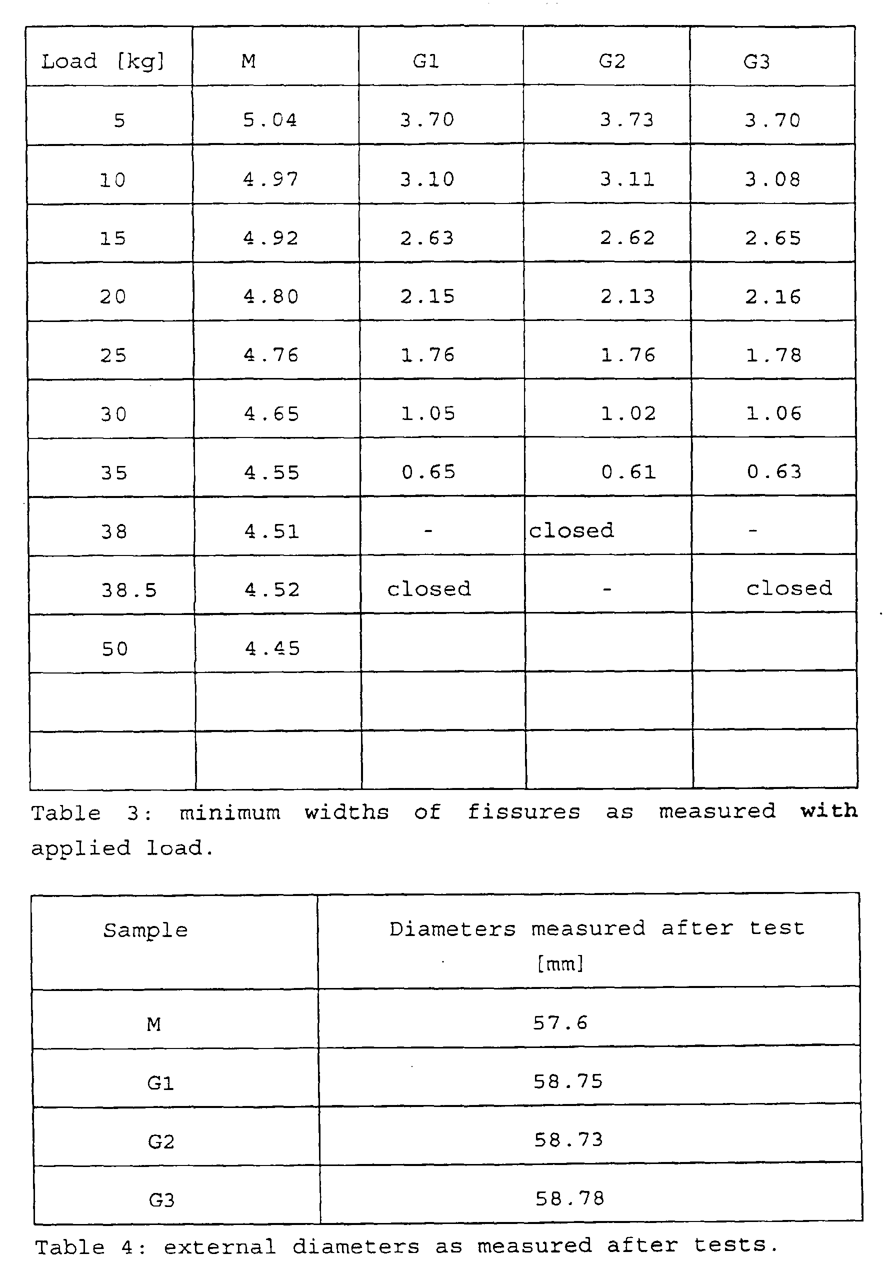

The results obtained show a very considerable elasticity in the calotte according to the present invention as compared with almost nonexistent elasticity in the conventional calotte of EP-A-0483023. This allows the cup according to the present invention to interact differently with the stages of implantation, providing a press-fit in two stages: an initial stage in which the calotte 1 is tapped into position and which is assisted by its considerable elasticity; followed by a stage of internal expansion during the insertion of the inner portion 17. The tests have also demonstrated that the deformations induced in the calotte of the cup according to the invention, although very considerable, remain within the strictly elastic range (cp. Table 4), meaning that dangerous plastic deformations are avoided during tapping.

The presence of any reference numerals in the accompanying claims is for the purpose of facilitating the reading of the claims with reference to the description and drawing, and does not restrict the scope of protection represented by the claims.

## Claims

1. Cup for the reconstruction of the acetabulum of the hip joint, comprising an outer calotte (1) intended for implantation in an acetabular cavity, and an inner portion (17) to be fitted inside said outer calotte, which said outer calotte (1) comprises a fissure (7) running from the apex region (5) to the edge (1C) of the calotte, and in which two tongues (9) are formed on the outer surface (1B) of the calotte (1), on either side of the fissure (7), wherein:
- the wall thickness of the calotte (1) is substantially uniform throughout said calotte, and comprised between 1,5 and 3 mm;
- the size of the tongues (9) and the wall thickness and material of the calotte (1) are such as to produce an elastic deformation of contraction as the calotte is tapped into the acetabular cavity, followed by expansion to the original size when the inner portion (17) is inserted; and
- said tongues (9) have such a radial extension that when the calotte (1) has been tapped home into the acetabular cavity and the inner portion (17) has been inserted into said calotte (1), the cup (1, 17) is firmly anchored to the bone.

2. Cup according to Claim 1, characterised in that the wall thickness of the calotte (1) is 2 mm.

3. Cup according to Claim 1 or 2, characterised in that said calotte (1) is a complete hemisphere.

4. Cup according to any of the preceding claims, characterised in that each of said tongues (9) extends from the base edge (1C) of the calotte (1), along a respective great circular line, towards the apex of the calotte.

5. Cup according to any of the previous claims, characterised in that each of said tongues (9) extends along the outer surface (1B) of the calotte (1) for at least one third of the height of the calotte.

6. Cup according to any of the previous claims, characterised in that each of said tongues comprises a substantially straight upper edge (9B) at an inclined angle to the polar axis (A-A) of the calotte (1).

7. Cup according to Claim 6, characterised in that said upper edge (9B) forms an angle of between 45° and 65° with said axis (AA), and preferably of 55°.

8. Cup according to any of the previous claims, characterised in that said tongues project radially from the outer surface (1B) of said calotte (1) by more than twice the thickness of the wall of the calotte (1).

9. Cup according to any of the previous claims, characterised in that on the inside of the calotte (1), adjacent to the latter's edge (1C), is an annular internal groove (13) in which are means (13A) for locking together said calotte (1) and said inner portion (17) in a particular relative angular position selected from among a plurality of possible relative positions.

10. Cup according to Claim 9, in which said means (13A) for the angular locking together of said calotte and of said inner portion comprise a plurality of undulating teeth.

11. Cup according to Claim 9 or 10, in which there is a second annular groove (19) on the inner surface of the calotte, and in that said inner portion (17) comprises an elastic annular projection (21) which, when the inner portion is placed in the calotte, snaps into place inside said second annular groove (19).

12. Cup according to any of the previous claims, characterised in that said tongues (9) join up with the outer surface of the calotte (1) along an edge that extends to an arc of between 15° and 25°, and preferably equal to 20°, in the plane (P) of the edge (1C) of said calotte (1).

## Patentansprüche

1. Verankerungsschale für die Wiederherstellung des Acetabuli des Hüftgelenks, bestehend aus einer äußeren Kalotte (1) vorgesehen für die Implantation in eine acetabulare Vertiefung, und einem inneren Teil (17) für die Befestigung in der äußeren Kalotte, wobei die äußere Kalotte (1) eine Spalte (7) umfaßt, die vom Scheitelbereich (5) zur Kante (1C) der Kalotte verläuft und an der zwei Zungen (9) auf der äußeren Oberfläche (1B) der Kalotte (1) auf beiden Seiten der Spalte (7) angeformt sind, wobei:
- die Wanddicke der Kalotte (1) im Wesentlichen im gesamten Bereich der Kalotte einheitlich ist und zwischen 1,5 und 3 mm umfaßt;
- die Größe der Zugen (9) und die Wanddicke und das Material der Kalotte (1) so sind, daß sie eine elastische Deformation bei der Kontraktion erzeugen, wenn die Kalotte in die acetabulare Vertiefung eingeklopft wird, der eine Ausdehnung auf die ursprüngliche Größe folgt, wenn der innere Teil (17) eingesetzt wird; und
- die Zungen (9) so eine radiale Ausdehnung haben, daß wenn die Kalotte (1) in die acetabulare Vertiefung eingeklopft ist und der innere Teil (17) in die Kalotte eingesetzt ist, die Verankerungsschale (1, 17) fest mit dem Knochen verankert ist.

2. Verankerungsschale gemäß Anspruch 1, dadurch gekennzeichnet, daß die Wanddicke der Kalotte (1) 2 mm beträgt.

3. Verankerungsschale gemäß der Ansprüche 1 oder 2, dadurch gekennzeichnet, daß die Kalotte (1) eine vollständige Halbkugel ist.

4. Verankerungsschale nach einem der vorhergehenden Ansprüche, dadurch gekennzeichnet, daß jede der Zungen (9) sich von der Grundkante (1C) der Kalotte (1) entlang einer jeweiligen großen Kreislinie zum Scheitelpunkt der Kalotte erstreckt.

5. Verankerungsschale gemäß einem der vorhergehenden Ansprüche, dadurch gekennzeichnet, daß jede der Zungen (9) sich entlang der äußeren Oberfläche (1B) der Kalotte (1) über wenigstens ein Drittel der Höhe der Kalotte erstreckt.

6. Verankerungsschale gemäß einem der vorhergehenden Ansprüche, dadurch gekennzeichnet, daß jede der Zungen eine im Wesentlichen gerade obere Kante (9B) in einem geneigten Winkel zu der polaren Achse (A-A) der Kalotte (1) umfaßt.

7. Verankerungsschale gemäß Anspruch 6, dadurch gekennzeichnet, daß die obere Kante (9B) einen Winkel zwischen 45 und 65 Grad mit der Achse (A-A) von vorzugsweise 55 Grad bildet.

8. Verankerungsschale entsprechend einem der vorhergehenden Ansprüche, dadurch gekennzeichnet, daß die Zungen sich radial von der äußeren Oberfläche (1B) der Kalotte (1) um mehr als die zweifache Dicke der Wand der Kalotte (1) erstrecken.

9. Verankerungsschale gemäß einem der vorhergehenden Ansprüche, dadurch gekennzeichnet, daß auf der Innenseite der Kalotte (1) benachbart zu der Kante (1C) eine ringförmige, innen liegende Rille (13) ist, in der Mittel (13A) für die Verbindung der Kalotte (1) und des inneren Teils (17) in einer besonderen relativen Winkelposition, die aus einer Vielzahl von möglichen relativen Positionen gewählt ist, angeordnet sind.

10. Verankerungsschale gemäß Anspruch 9, bei der die Mittel (13A) für die winkelfeste Verbindung der Kalotte und des inneren Teils eine Vielzahl von wellenförmigenn Zähnen umfassen.

11. Verankerungsschale gemäß den Ansprüchen 9 oder 10, bei der auf der inneren Oberfläche der Kalotte eine zweite ringförmige Rille (19) angeordnet ist, wobei der innere Teil (17) einen elastischen ringförmigen Vorsprung (21) umfaßt, der, wenn der innere Teil in die Kalotte gesetzt wird, auf der Innenseite der zweiten ringförmigen Rille (19) einschnappt.

12. Verankerungsschale gemäß einem der vorhergehenden Ansprüche, dadurch gekennzeichnet, daß die Zungen (9) in die äußere Oberfläche der Kalotte (1) entlang einer Kante, die sich über einen Bogen von zwischen 15° und 25°, vorzugsweise 20°, in der Ebene (P) der Kante (1C) der Kalotte (1) erstreckt, übergehen.

## Revendications

1. Coquille de fixation pour la reconstruction du cotyle de hanche comprenant une calotte extérieure (1) destinée à être implantée dans une cavité cotyloïde et une partie intérieure (17) destinée à être adaptée à l'intérieur de ladite calotte extérieure, laquelle calotte extérieure(1) comprend une fente (7) s'étendant de la région apicale (5) au bord (1C) de la calotte et dans laquelle deux languettes (9) sont formées sur la surface extérieure (1B) de la calotte (1) sur l'un ou l'autre côté de la fente (7), dans laquelle :
- l'épaisseur de paroi de la calotte (1) est essentiellement uniforme au travers de ladite calotte et comprise entre 1,5 et 3 mm ;
- la dimension des languettes (9) et l'épaisseur de paroi et le matériau de la calotte (1) sont tels qu'ils produisent une déformation élastique de contraction lorsque la calotte est conformée dans la cavité cotyloïde suivie par une dilatation à la dimension d'origine lorsque la partie intérieure (17) est insérée ; et
- lesdites languettes (9) ont une telle extension radiale que lorsque la calotte (1) a été ramenée dans la cavité cotyloïde et que la partie intérieure (17) a été insérée dans ladite calotte (1), la coquille de fixation (1,17) est fermement ancrée dans l'os.

2. Coquille de fixation selon la revendication 1, caractérisée en ce que l'épaisseur de paroi de la calotte (1) est de 2 mm.

3. Coquille de fixation selon la revendication 1 ou 2, caractérisée en ce que ladite calotte (1) est un hémisphère complet.

4. Coquille de fixation selon l'une quelconque des revendications précédentes, caractérisée en ce que chacune desdites languettes (9) se prolonge du bord de base (1C) de la calotte (1) le long d'une grande ligne circulaire respective vers la partie apicale de la calotte.

5. Coquille de fixation selon l'une quelconque des revendications précédentes, caractérisée en ce que chacune desdites languettes (9) se prolonge le long de la surface extérieure (1B) de la calotte (1) pour au moins un tiers de la hauteur de la calotte.

6. Coquille de fixation selon l'une quelconque des revendications précédentes, caractérisée en ce que chacune desdites languettes comporte un bord supérieur sensiblement droit (9B) selon un angle incliné par rapport à l'axe polaire (A-A) de la calotte (1).

7. Coquille de fixation selon la revendication 6, caractérisée en ce que ledit bord supérieur (9B) forme un angle compris entre 45° et 65° avec ledit axe (A-A) et de préférence de 55°.

8. Coquille de fixation selon l'une quelconque des revendications précédentes, caractérisée en ce que lesdites languettes font saillie radialement à partir de la surface extérieure (1B) de ladite calotte de plus de deux fois l'épaisseur de la paroi de la calotte (1).

9. Coquille de fixation selon l'une quelconque des revendications précédentes, caractérisée en ce que sur l'intérieur de la calotte (1), près du bord de cette dernière (1C) se trouve une gorge interne annulaire (13) dans laquelle il y a des moyens (13A) pour bloquer conjointement ladite calotte (1) et ladite partie intérieure (17) dans une position angulaire relative particulière choisie parmi une pluralité de positions relatives possibles.

10. Coquille de fixation selon la revendication 9, dans laquelle lesdits moyens (13A) pour le blocage angulaire conjointement de ladite calotte et de ladite partie intérieure comprennent une pluralité de dents à ondulation.

11. Coquille de fixation selon la revendication 9 ou 10, dans laquelle il y a une deuxième gorge annulaire (19) sur la surface intérieure de la calotte et dans laquelle ladite partie intérieure (17) comprend un prolongement annulaire élastique (21) qui lorsque la partie intérieure est placée dans la calotte revient en place à l'intérieur de ladite seconde gorge annulaire (19).

12. Coquille de fixation selon l'une quelconque des revendications précédentes, caractérisée en ce que lesdites languettes (9) rejoignent la surface extérieure de la calotte (1) le long d'un bord qui s'étend selon un arc entre 15° et 25° et qui est de préférence égal à 20° dans le plan (P) du bord (1C) de ladite calotte (1).
